# EUROPEAN PATENT APPLICATION

(11) **EP 1 516 580 A1**
(43) Date of publication of application: **23.03.2005**
(21) Application number: 04021910.7
(22) Date of filing: 14.09.2004
(51) Int. Cl.: A61B 5/024, A61B 5/022

(54) **Wrist securing device for pulse wave measuring apparatus**

(30) Priority: 17.09.2003 JP 2003324917
(71) Applicant: Omron Healthcare Co., Ltd., Kyoto 615-0084 (JP)
(72) Inventor: Hashimoto, Masao c/o Omron Healthcare Co. Ltd., Kyoto-shi, Kyoto 615-0084 (JP); Itonaga, Kazunobo c/o Omron Healthcare Co. Ltd., Kyoto-shi, Kyoto 615-0084 (JP)
(74) Representative: Kilian, Helmut, Dr.

(57) **Abstract**

A wrist securing device (10A) for a pulse wave measuring apparatus includes a securing base main body (11) mounted on a mount surface, and a holding portion (20A) provided in an upper portion of the securing base main body (11) and receiving a wrist with a palm side portion of the wrist facing upward. The holding portion (20A) has a support surface (21) receiving the wrist in an inclined position so that an end portion on a radial side of the wrist is located above an end portion on an ulnar side thereof while the wrist rests on the holding portion. With such a structure, a wrist securing device for a pulse wave measuring apparatus capable of holding a wrist position while a subject maintains a natural posture can be implemented.

## Description

### BACKGROUND OF THE INVENTION

### Field of the Invention

The present invention relates to a wrist securing device for a pulse wave measuring apparatus holding a wrist position in measuring an intra-arterial pressure of a radial artery located under the skin of the wrist, as well as to a pulse wave measuring apparatus equipped with the same.

### Description of the Background Art

In many cases, circulatory diseases such as ischemic heart disease or stroke are induced by hypertension, arteriosclerosis, cardiac hypertrophy, or the like. Accordingly, in addition to simply keeping track of a blood pressure level of a patient, it is very important to keep track of load imposed on the heart or variation in hardening of the wall of an artery. A pulse wave measuring apparatus is known as an apparatus to observe such load imposed on the heart or variation in hardening of the wall of an artery.

The pulse wave measuring apparatus is an apparatus used for measuring a pulse wave by non-invasive detection of an intra-arterial pressure of the radial artery located in a relatively shallow portion under the skin of the wrist. Here, the pulse wave refers to periodic fluctuation of a pressure produced in a blood vessel in accordance with heart beat. From analysis of the pulse wave, the load imposed on the heart or variation in hardening of the wall of the artery can be grasped. Therefore, in order to know medical condition of a subject, it is extremely important to measure his/her pulse wave using the pulse wave measuring apparatus.

In the pulse wave measuring apparatus for measuring the pulse wave in a non-invasive manner, a semiconductor pressure sensor utilizing a strain gauge or a diaphragm is commonly used as pressure-sensing means. Here, the apparatus is configured such that the pressure-sensing means for detecting the intra-arterial pressure is located on a surface of a sensor unit attached on a wrist.

In order to measure the pulse wave with high accuracy, it is important to attach the sensor unit at an appropriate position on the wrist. In addition, it is essential to hold a wrist position in a stable manner during measurement. Accordingly, in the pulse wave measuring apparatus, a wrist securing device for holding the position of the wrist of the subject is normally used.

A wrist securing device for a pulse wave measuring apparatus for holding the position of the wrist is disclosed, for example, in Japanese Utility Model Laying-Open No. 3-67605, Japanese Patent Laying-Open Nos. 5-261074 and 11-33007, or the like. The wrist securing device for a pulse wave measuring apparatus disclosed in these publications serves to hold the position of the wrist of the subject using a plate-shaped member and a band attached thereto. In the following, the wrist securing device for a pulse wave measuring apparatus disclosed in Japanese Patent Laying-Open No. 11-33007 will be described by way of example, so as to explain a structure of a conventional wrist securing device for a pulse wave measuring apparatus.

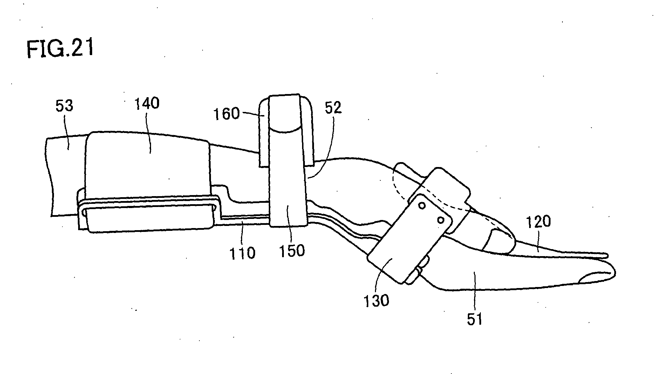

Fig. 21 shows a structure of a conventional wrist securing device for a pulse wave measuring apparatus disclosed in Japanese Patent Laying-Open No. 11-33007. The wrist securing device for a pulse wave measuring apparatus shown in Fig. 21 includes a plate-shaped outer abutment member 110 being in contact with the outer side of a hand 51, a wrist 52 and a forearm 53 and curved so as to secure wrist 52 in a state in which wrist 52 is bent toward a dorsal side; a plate-shaped inner abutment member 120 being in intimate contact with the inner side of fingers and palm of hand 51; a fastening band 130 connecting outer abutment member 110 and inner abutment member 120; and an attachment band 140 for attaching outer abutment member 110 to forearm 53. In a state in which the wrist securing device for a pulse wave measuring apparatus is attached on the subject as shown in Fig. 21, a sensor unit 160 is attached on a palm side portion of wrist 52 using an attachment band 150, so as to perform measurement of the pulse wave. The wrist position is stabilized by using the wrist securing device for a pulse wave measuring apparatus, so that the sensor unit can be attached right above the radial artery with higher accuracy.

On the other hand, though the wrist position is held in a stable manner with the wrist securing device for a pulse wave measuring apparatus described above, attachment of the wrist securing device is considerably complicated. In addition, since not only the wrist but also the hand and the forearm are secured in order to hold the wrist position in the wrist securing device for a pulse wave measuring apparatus described above, the subject may feel great pain.

### SUMMARY OF THE INVENTION

In general, in order to measure the pulse wave, a measurement time from several minutes to over ten minutes is necessary. Therefore, it is important to structure the wrist securing device for a pulse wave measuring apparatus for holding the wrist position such that a natural posture of the subject can be maintained without causing pain to him/her. With such a structure, body motion of the subject during measurement is suppressed. Then, the pulse wave can be measured in a stable manner with high accuracy, and an appropriate measurement result can be obtained.

From the foregoing, an object of the present invention is to provide a wrist securing device for a pulse wave measuring apparatus capable of holding a wrist position while maintaining a natural posture of a subject. In addition, it is also an object of the present invention to provide a pulse wave measuring apparatus capable of stable measurement of a pulse wave with high accuracy by including such a wrist securing device for a pulse wave measuring apparatus.

A wrist securing device for a pulse wave measuring apparatus according to a first aspect of the present invention includes a securing base main body mounted on a mount surface, and a holding portion provided in an upper portion of the securing base main body and receiving a wrist with a palm side portion of the wrist facing upward. The wrist securing device for a pulse wave measuring apparatus holds the position of the wrist placed on the holding portion for measurement of periodic fluctuation of an intra-arterial pressure of a radial artery located under the skin of the wrist. In the wrist securing device for a pulse wave measuring apparatus, the holding portion has a support surface receiving the wrist in an inclined position so that an end portion on a radial side of the wrist is located above an end portion on an ulnar side thereof while the wrist rests on the holding portion.

A wrist securing device for a pulse wave measuring apparatus according to a second aspect of the present invention includes a securing base main body mounted on a mount surface, and a holding portion provided in an upper portion of the securing base main body and receiving a wrist with a palm side portion of the wrist facing upward. The wrist securing device for a pulse wave measuring apparatus holds the position of the wrist placed on the holding portion for measurement of periodic fluctuation of an intra-arterial pressure of a radial artery located under the skin of the wrist. The holding portion has a support surface receiving a dorsal side portion of the wrist while the wrist rests on the holding portion. The support surface includes a first support region receiving a radial side portion of the wrist and a second support region receiving an ulnar side portion of the wrist. In the wrist securing device for a pulse wave measuring apparatus, the support surface in a position corresponding to the first support region is structured to incline more gently than the support surface in a position corresponding to the second support region.

A wrist securing device for a pulse wave measuring apparatus according to a third aspect of the present invention includes a securing base main body mounted on a mount surface, and a holding portion provided in an upper portion of the securing base main body and receiving a wrist with a palm side portion of the wrist facing upward. The wrist securing device for a pulse wave measuring apparatus holds the position of the wrist placed on the holding portion for measurement of periodic fluctuation of an intra-arterial pressure of a radial artery located under the skin of the wrist. The holding portion has a support surface receiving a dorsal side portion of the wrist while the wrist rests on the holding portion. The support surface includes a first support region receiving a radial side portion of the wrist and a second support region receiving an ulnar side portion of the wrist. In the wrist securing device for a pulse wave measuring apparatus, the first support region extends above the second support region.

A wrist securing device for a pulse wave measuring apparatus according to a fourth aspect of the present invention includes a securing base main body mounted on a mount surface, and a holding portion provided in an upper portion of the securing base main body and receiving a wrist with a palm side portion of the wrist facing upward. The wrist securing device for a pulse wave measuring apparatus holds the position of the wrist placed on the holding portion for measurement of periodic fluctuation of an intra-arterial pressure of the radial artery located under the skin of the wrist. The holding portion has a support surface receiving a dorsal side portion of the wrist while the wrist rests on the holding portion. The support surface includes a first support region receiving a radial side portion of the wrist and a second support region receiving an ulnar side portion of the wrist. In the wrist securing device for a pulse wave measuring apparatus, the support surface is configured to be asymmetrical around a vertical centerline including a central portion of the support surface in a cross section along a direction in which the first support region and the second support region are aligned.

In the wrist securing devices for a pulse wave measuring apparatus according to the first to fourth aspects of the present invention described above, preferably, the holding portion has a concave portion in a position corresponding to an ulnar styloid process of the wrist while the wrist rests on the holding portion.

In the wrist securing devices for a pulse wave measuring apparatus according to the first to fourth aspects of the present invention described above, preferably, the holding portion has a buffer portion composed of an elastic member in a position corresponding to an ulnar styloid process of the wrist while the wrist rests on the holding portion.

In the wrist securing devices for a pulse wave measuring apparatus according to the first to fourth aspects of the present invention described above, preferably, the holding portion has a right wrist receiving region and a left wrist receiving region. Here, preferably, the holding portion is configured so as to be switchable between a first state allowing use of the right wrist receiving region and a second state allowing use of the left wrist receiving region. In order to realize switching between the states described above, preferably, the holding portion is attached to the securing base main body in a detachable manner or in a swingable manner.

In the wrist securing devices for a pulse wave measuring apparatus according to the first to fourth aspects of the present invention described above, preferably, the holding portion has a positioning marker in a position corresponding to wrist joint anterior surface stripe located on an outer surface of the wrist while the wrist rests on the holding portion.

The pulse wave measuring apparatus according to the present invention includes a sensor unit having a pressure-sensing portion and a living body securing device holding a wrist position, and serves to measure the pulse wave by pressing the pressure-sensing portion against the wrist while the wrist is secured by the living body securing device. The living body securing device includes any of the wrist securing devices for a pulse wave measuring apparatus described above.

With the wrist securing devices for a pulse wave measuring apparatus according to the first to fourth aspects of the present invention described above, the wrist position can be held while a natural posture of the subject is maintained. Accordingly, the pulse wave measuring apparatus equipped with the wrist securing device for a pulse wave measuring apparatus as described above is implemented, and stable measurement of the pulse wave with high accuracy is allowed.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic perspective view showing an overall structure of a pulse wave measuring apparatus including a wrist securing device for a pulse wave measuring apparatus in the related art.
Fig. 2 is a schematic cross sectional view including a holding portion, without showing an internal mechanism of the pulse wave measuring apparatus shown in Fig. 1.
Fig. 3 is a schematic perspective view showing a left arm placed in the pulse wave measuring apparatus shown in Fig. 1.
Fig. 4A is a skeletal view from a palm side of the left hand of a human body.
Fig. 4B is a cross sectional view of the left wrist along the line IVB-IVB in Fig. 4A.
Fig. 5 is a cross sectional view of the left wrist when measurement is performed with the left arm, illustrating a measurement position of the wrist conceived by the inventor.
Fig. 6 is a cross sectional view of the right wrist when measurement is performed with the right arm, illustrating a measurement position of the wrist conceived by the inventor.
Fig. 7 is a schematic perspective view showing an overall structure of a pulse wave measuring apparatus equipped with a wrist securing device for a pulse wave measuring apparatus in a first embodiment.
Fig. 8 is a schematic perspective view of a holding portion of the wrist securing device for a pulse wave measuring apparatus in the first embodiment.
Fig. 9 is a schematic vertical cross sectional view including a holding portion, without showing an internal mechanism of the wrist securing device for a pulse wave measuring apparatus shown in the first embodiment.
Fig. 10 is a schematic vertical cross sectional view including the holding portion of the wrist securing device for a pulse wave measuring apparatus in the first embodiment.
Figs. 11 and 12 are schematic vertical cross sectional views illustrating a procedure for attaching the pulse wave measuring apparatus in the first embodiment.
Fig. 13 is a schematic perspective view showing an attached state of the pulse wave measuring apparatus in the first embodiment.
Fig. 14 is a schematic vertical cross sectional view illustrating a measurement operation with the pulse wave measuring apparatus in the first embodiment.
Fig. 15 is a schematic vertical cross sectional view of the holding portion of the wrist securing device for a pulse wave measuring apparatus in the first embodiment.
Fig. 16 is a plan view of the holding portion of the wrist, securing device for a pulse wave measuring apparatus in the first embodiment.
Figs. 17A to 17C are schematic diagrams showing a manner of attaching/removing the holding portion to/from a securing base main body in the wrist securing device for a pulse wave measuring apparatus in the first embodiment.
Fig. 18 is a schematic perspective view showing that a subject places the right arm in the pulse wave measuring apparatus in the first embodiment.
Fig. 19 is a cross sectional view including a holding portion, of a wrist securing device for a pulse wave measuring apparatus and the pulse wave measuring apparatus equipped with the same in a second embodiment.
Fig. 20A is a schematic vertical cross sectional view showing a structure of a holding portion of a wrist securing device for a pulse wave measuring apparatus in a third embodiment, and showing a first state in which the left wrist rests on the holding portion.
Fig. 20B is a schematic vertical cross sectional view showing the structure of the holding portion of the wrist securing device for a pulse wave measuring apparatus in the third embodiment, and showing a second state in which the right wrist rests on the holding portion.
Fig. 21 illustrates a structure of a conventional wrist securing device for a pulse wave measuring apparatus.

### DESCRIPTION OF THE PREFERRED EMBODIMENTS

The inventor conceived that a wrist securing device for a pulse wave measuring apparatus is configured so as to allow a natural posture of a subject while the pulse wave is measured with the pulse wave measuring apparatus, whereby body motion of the subject during measurement is suppressed and the pulse wave is measured in a stable manner with high accuracy. Then, the inventor has developed a wrist securing device for a pulse wave measuring apparatus for maintaining a wrist position in a natural posture, instead of maintaining the wrist position of the subject by attaching the attachment band on the hand or the forearm of the subject as in the conventional wrist securing device for a pulse wave measuring apparatus. Initially, a wrist securing device for a pulse wave measuring apparatus invented in the course of development above will be described.

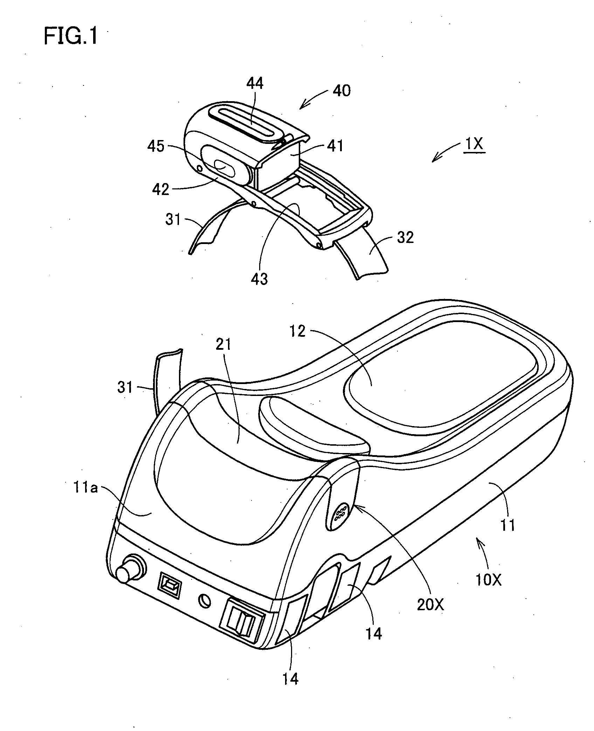

As shown in Fig. 1, a pulse wave measuring apparatus 1X in the related art of the present invention mainly includes a sensor unit 40, a wrist securing device 10X for a pulse wave measuring apparatus serving as a living body securing device, and fastening bands 31, 32.

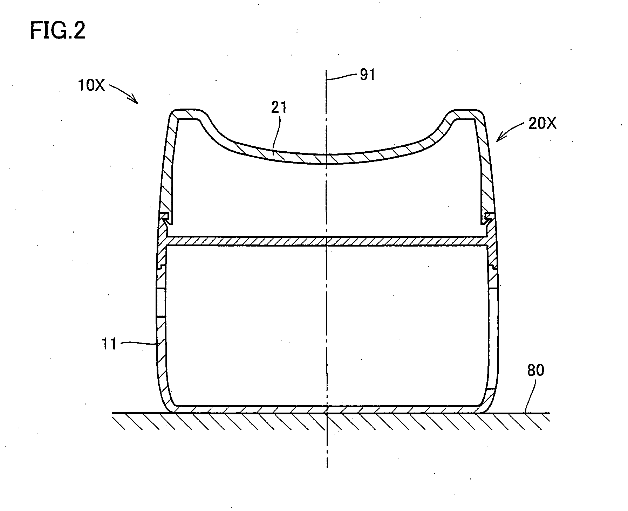

Wrist securing device 10X for a pulse wave measuring apparatus is placed on a flat base such as a desk during use. Wrist securing device 10X for a pulse wave measuring apparatus includes a securing base main body 11 mounted on a mount surface 80 (see Fig. 2) and a holding portion 20X provided in an upper portion of securing base main body 11. In a front face 11a of securing base main body 11, an operation button or an output terminal is provided. Here, the output terminal refers to a terminal for transmitting pulse wave data detected by the pulse wave measuring apparatus to a processor terminal such as an external PC (Personal Computer).

In the upper portion of securing base main body 11, a concave portion 12 capable of receiving an arm from wrist to elbow of the subject is provided. Concave portion 12 is structured so as to have a shape fitting to the arm from the wrist to the elbow of the subject, for example. In order to ease pain caused by direct contact of the arm of the subject to securing base main body 11, preferably, a buffer material such as a cushion material made from sponge, for example, is adhered to the surface of concave portion 12. Alternatively, a structure solely having a buffer material adhered without a concave portion in the upper portion of securing base main body 11 may be possible.

As shown in Fig. 2, holding portion 20X has a support surface 21 receiving the wrist on the upper portion. Support surface 21 is shaped symmetrically around a centerline 91 of holding portion 20X in a cross section orthogonal to a longitudinal direction of securing base main body 11, so that support surface 21 can receive any of left and right wrists. It is noted that an internal mechanism of the securing base main body is not shown in Fig. 2.

As shown in Fig. 1, sensor unit 40 includes a casing body 41 having a pressure-sensing portion (not shown) implemented by a semiconductor pressure sensor and a base body 42 supporting casing body 41. Casing body 41 is structured such that it can slide on a rail provided in base body 42 in a longitudinal direction of fastening bands 31 and 32 described later. In other words, casing body 41 freely slides between a position closing an opening 43 provided in base body 42 (waiting position) and a position not closing the same (housed position). Fig. 1 shows casing body 41 in a housed position. When casing body 41 is slid, casing body 41 is moved with an engagement release button 45 provided on a side surface of casing body 41 being pressed.

Opening 43 is provided such that the pressure-sensing portion arranged inside casing body 41 can be pressed against the wrist on which measurement is performed. When the pressure-sensing portion is lowered through opening 43, the pressure-sensing portion is pressed against the wrist so as to allow measurement of the pulse wave. In addition, a display portion 44 for displaying whether or not the pressure-sensing portion during measurement is in an appropriate position is provided on an upper surface of sensor unit 40.

Securing base main body 11 and sensor unit 40 are connected to each other by fastening bands 31 and 32. The fastening band is implemented by a first band portion 31 having one end attached to base body 42 of sensor unit 40 and the other end attached to securing base main body 11 and a second band portion 32 having one end attached to base body 42 of sensor unit 40 and the other end detachably attached to securing base main body 11. Here, each band is moderately flexible.

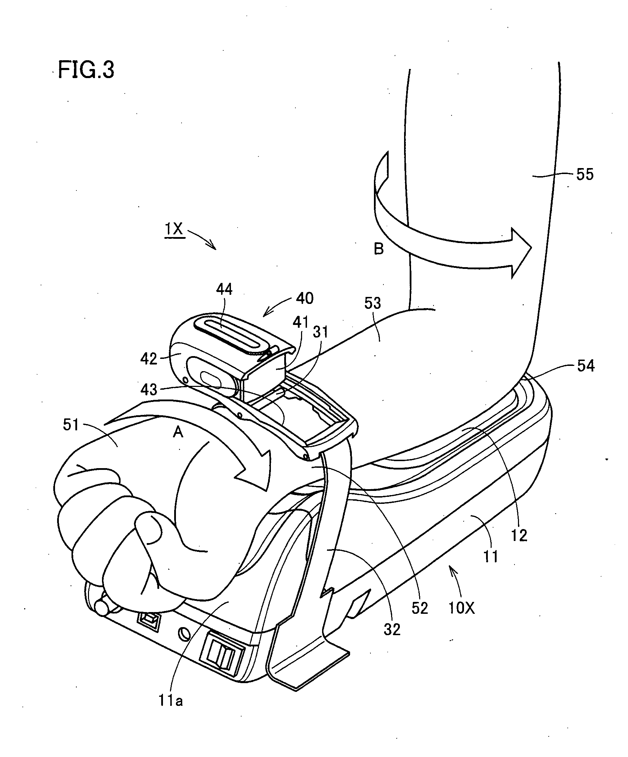

As shown in Fig. 3, in measuring the pulse wave, the left arm is placed such that the arm from elbow 54 to forearm 53 is placed on concave portion 12 of securing base main body 11, and wrist 52 is placed on support surface 21 of holding portion 20X. Then, first band portion 31 is drawn out of securing base main body 11, sensor unit 40 is arranged so as to be positioned right above wrist 52 of the subject, and second band portion 32 is attached on a side surface of securing base main body 11 opposite to the side surface from which first band portion 31 is drawn out. Here, for attachment of second band portion 32 to securing base main body 11, a hook and loop fastener 14 (see Fig. 1) adhered on the side surface of securing base main body 11 and a hook and loop fastener (not shown) adhered on an inner side of a tip end of the other end of second band portion 32 are used.

According to the pulse wave measuring apparatus equipped with the wrist securing device for a pulse wave measuring apparatus of the structure as described above, the wrist position can be fixed while the subject takes a natural posture without the hand or forearm being fastened. Therefore, the pulse wave can be measured without causing pain to the subject due to fastening the band around the hand or arm.

With the pulse wave measuring apparatus having the structure as described above, the hand or forearm is no longer forcibly secured by a securing device. Though the subject is now free from pain due to fastening, it has been found that following problems still remain.

First, a brachium may be twisted. As described above, in wrist securing device 10X for a pulse wave measuring apparatus shown in Figs. 1 to 3, in order to receive any of the left and right arms, the shape of support surface 21 in the cross section orthogonal to the longitudinal direction of securing base main body 11 is symmetrical around centerline 91 (see Fig. 2). Accordingly, when the subject places the arm on securing base main body 11, he/she has to face the palm side portion of wrist 52 upward by twisting wrist 52 by approximately 90° in a direction shown with an arrow A in Fig. 3. Then, brachium 55 also needs to be twisted in a direction shown with an arrow B in the figure. In general, in order to measure the pulse wave, such a posture should be maintained for a time period from several minutes to over ten minutes. Maintaining such a posture, however, may cause pain to the subject. In particular, muscle of the brachium of elderly people tends to be rigid due to aging in many cases, and they are likely to twist the wrist in the opposite direction during measurement in order to ease pain, resulting in difficulty in stable and accurate measurement of the pulse wave.

Secondly, pain may be caused on the dorsal side portion of the wrist corresponding to an ulnar styloid process. In wrist securing device 10X for a pulse wave measuring apparatus described above, as the dorsal side portion of wrist 52 corresponding to the ulnar styloid process comes in direct contact with support surface 21, the subject may feel pain. Accordingly, with wrist securing device 10X for a pulse wave measuring apparatus having the above-described structure, it is likely that the subject may move wrist 52 during measurement in order to ease pain, resulting in difficulty in stable and accurate measurement of the pulse wave.

Thirdly, it is difficult to appropriately position and place wrist 52 on holding portion 20X. In wrist securing device 10X for a pulse wave measuring apparatus described above, as elbow 54 should be placed on concave portion 12 provided in the upper portion of securing base main body 11, it is expected that the subject first places his/her elbow 54, and thereafter places wrist 52 on holding portion 20X. If.elbow 54 is not placed in an appropriate position, wrist 52 is not appropriately positioned on holding portion 20X either, resulting in need for positioning again.

The inventor furthered development in order to solve the above-described problems, and completed the present invention. In the following, embodiments of the present invention will be described with reference to the drawings.

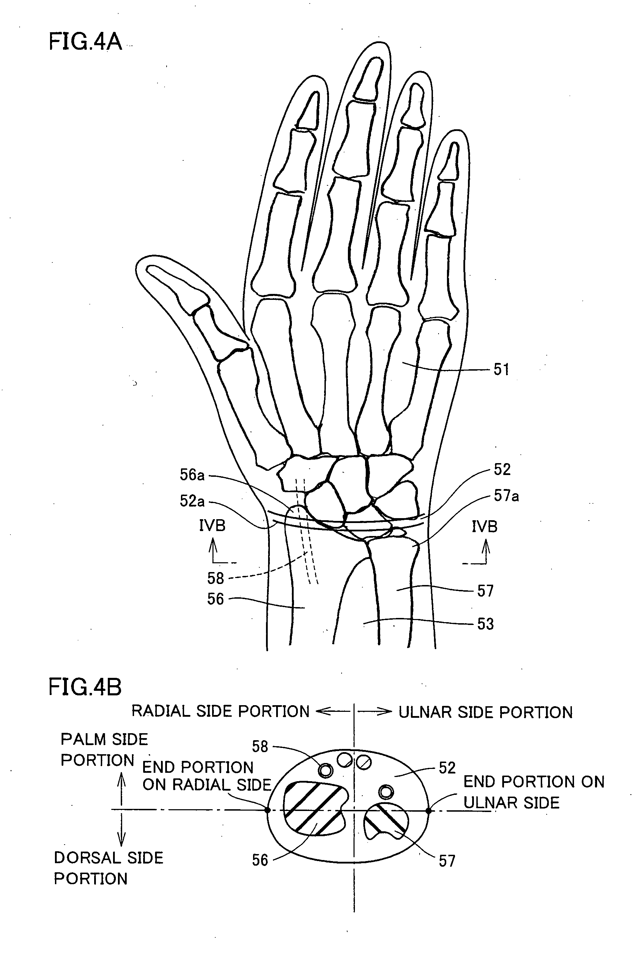

Referring first to Figs. 4A and 4B, a structure of a hand of a human body will be described and definition of terms used herein will be provided.

As shown in Figs. 4A and 4B, radius 56 and ulna 57 are located in wrist 52, extending between hand 51 and forearm 53. Radius 56 and ulna 57 are located parallel to each other from the elbow to wrist 52, and end portions of both of the radius and the ulna are located in wrist 52. The respective end portions of radius 56 and ulna 57 have protrusions referred to as radial styloid process 56a and ulnar styloid process 57a respectively. A radial artery 58 is located between radius 56 and the skin on the palm side of wrist 52. Wrist joint anterior surface stripe 52a is located on an outer surface on the palm side of wrist 52.

As shown in Fig. 4B, in the present specification, the palm side of wrist 52 is referred to as the palm side portion, the dorsal side thereof is referred to as the dorsal side portion, radial 56 side thereof is referred to as the radial side portion, and ulnar 57 side thereof is referred to as the ulnar side portion. In addition, of boundaries between the palm side portion and the dorsal side portion of the wrist on a body surface, a portion located on the body surface of the radial side portion is referred to as an end portion on the radial side, and a portion located on the body surface of the ulnar side portion is referred to as an end portion on the ulnar side.

The inventor conceived that a shape of a mount surface of the holding portion should be varied in order to allow a posture for measurement closer to natural for the subject. Normally, the palm side portion of the wrist faces the body in a natural posture. Therefore, the inventor assumed that the wrist is preferably positioned in a manner inclined somewhat diagonally with respect to the body during measurement, instead of the palm side portion of the wrist facing upward, considering a posture for measurement closer to natural.

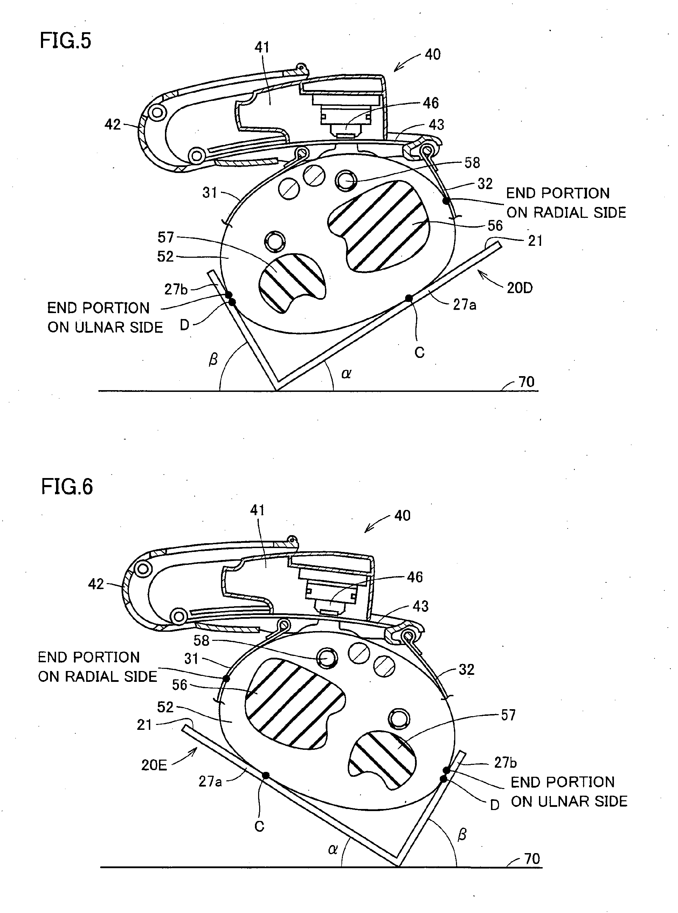

In the following, the wrist position during measurement conceived by the inventor will be described with reference to Figs. 5 and 6. Figs. 5 and 6 both show cross sections of the wrist viewed from a peripheral side toward a central side.

As shown in Fig. 5, when measurement is performed on the left wrist, wrist 52 is inclined toward the body, that is, in such a manner that the palm side thereof faces the body, so that the end portion on the radial side of wrist 52 is located above the end portion on the ulnar side of the same. When wrist 52 is inclined such that the end portion on the radial side of wrist 52 is located above the end portion on the ulnar side of wrist 52, twisting of the brachium is mitigated and the subject can take a more natural posture.

One exemplary shape of the holding portion allowing such a wrist position is as shown in Fig. 5. A holding portion 20D shown in Fig. 5 has support surface 21 receiving the dorsal side portion of wrist 52, and support surface 21 includes a first support region 27a receiving the radial side portion of wrist 52 and a second support region 27b receiving the ulnar side portion of wrist 52. Here, support surface 21, first support region 27a, and second support region 27b are designed taking into account individual difference in a size of the wrist of the subject, and they refer to regions intended to support wrist 52 when the subject actually places his/her wrist 52 on holding portion 20D respectively.

In holding portion 20D shown in Fig. 5, first support region 27a and second support region 27b both have planar shape, and come in contact with the radial side portion and the ulnar side portion of wrist 52 at points C and D respectively.

In shown holding portion 20D, the support surface in first support region 27a is inclined more gently than the support surface in second support region 27b. That is, when an angle α between a horizontal surface 70 and the support surface in first support region 27a is compared with an angle β between horizontal surface 70 and the support surface in second support region 27b, angle β is larger than angle α.

In addition, in shown holding portion 20D, first support region 27a extends above second support region 27b. That is, in order to receive a wrist having an oval cross sectional shape, first support region 27a is formed to have a length larger than second support region 27b. As a result, first support region 27a extends higher than second support region 27b.

Moreover, in shown holding portion 20D, support surface 21 is formed asymmetrically around vertical centerline 91 including the central portion of support surface 21 in a cross section along a direction in which first support region 27a and second support region 27b are aligned (that is, shown cross section). In other words, support surface 21 is formed to have an asymmetrical shape so that the end portion on the radial side of left wrist 52 having an oval cross sectional shape is located above the end portion on the ulnar side thereof.

By structuring holding portion 20D in the above-described manner, when the left arm is placed in holding portion 20D, a wrist 52 position can be guided such that the end portion on the radial side of wrist 52 is located above the end portion on the ulnar side thereof. Therefore, a posture for measurement closer to natural for the subject can be allowed, and the pulse wave can be measured in a stable manner with high accuracy. In addition, as a result of adopting such a posture for measurement, radial artery 58 is located in the upper portion of placed wrist 52. Accordingly, positioning of sensor unit 40 is facilitated, and pressure-sensing portion 46 can readily be set right above radial artery 58.

Further, as shown in Fig. 6, when measurement is performed on right wrist 52 as well, wrist 52 is inclined toward the body, that is, in such a manner that the palm side faces the body, so that the end portion on the radial side of wrist 52 is located above the end portion on the ulnar side of wrist 52. In this manner, when wrist 52 is inclined such that the end portion on the radial side of wrist 52 is located above the end portion on the ulnar side of wrist 52, twisting of the brachium is mitigated and the subject can take a more natural posture.

One exemplary shape of the holding portion allowing such a wrist position is as shown in Fig. 6. A holding portion 20E of a shape shown in Fig. 6 is structured symmetrically to holding portion 20A for the left arm described above. Since its structure and an effect therefrom are substantially the same as in holding portion 20D, description thereof will not be repeated.

As described above, a posture for measurement with the wrist being inclined such that the end portion on the radial side of the wrist is located above the end portion of the ulnar side thereof is adopted. Accordingly, the pulse wave can be measured in a stable manner with high accuracy without causing pain to the subject. The structure of the holding portion of the wrist securing device for a pulse wave measuring apparatus allowing such a posture for measurement is not limited to the structure as those shown in Figs. 5 and 6. In the following, several other specific examples of the wrist securing device for a pulse wave measuring apparatus allowing the above-described posture for measurement as well as the pulse wave measuring apparatus equipped with the same will be described.

### [First Embodiment]

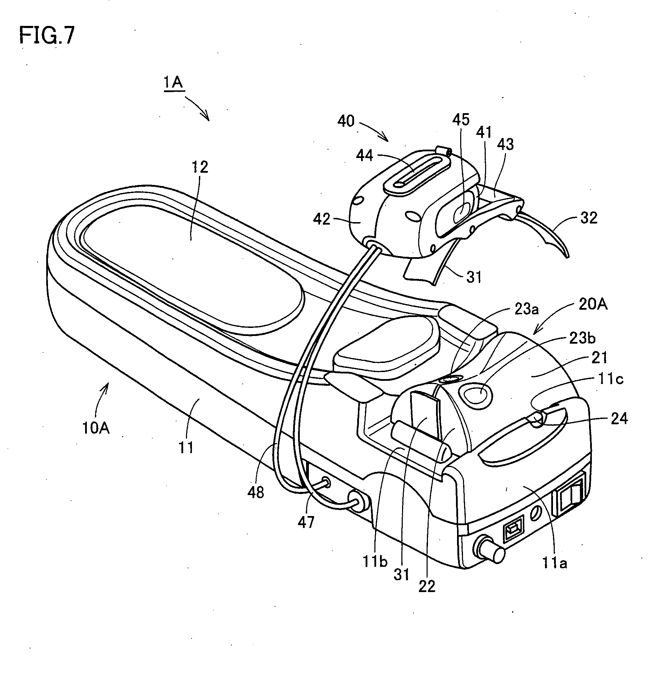

As shown in Fig. 7, a pulse wave measuring apparatus 1A in a first embodiment mainly includes sensor unit 40, a wrist securing device 10A for a pulse wave measuring apparatus serving as a living body securing device, and fastening bands 31, 32. Sensor unit 40 is connected to wrist securing device 10A for a pulse wave measuring apparatus by fastening bands 31 and 32. The fastening band is implemented by first band portion 31 having one end attached to sensor unit 40 and the other end attached to wrist securing device 10A for a pulse wave measuring apparatus and by second band portion 32 having one end attached to sensor unit 40 and the other end detachably attached to wrist securing device 10A for a pulse wave measuring apparatus. Here, each band is moderately flexible. In addition, sensor unit 40 and wrist securing device 10A for a pulse wave measuring apparatus are connected to each other by a signal cable 47 and an air tube 48.

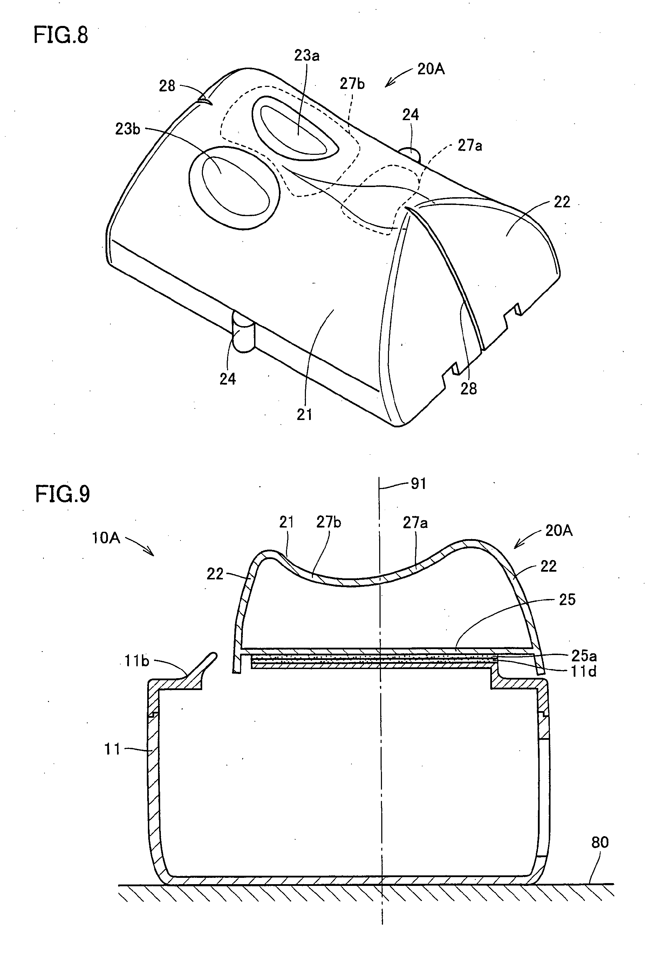

Wrist securing device 10A for a pulse wave measuring apparatus is placed on a flat base such as a desk during use. Wrist securing device 10A for a pulse wave measuring apparatus includes securing base main body 11 mounted on a mount surface 80 (see Fig. 9) and a holding portion 20A provided in an upper portion of securing base main body 11. In front face 11 a of securing base main body 11, an operation button or an output terminal is provided.

In the upper portion of securing base main body 11, concave portion 12 capable of receiving an arm from wrist to elbow of the subject and a concave portion 11b to which holding portion 20A is fitted are provided. Concave portion 12 is structured to have a shape fitting to the arm from the wrist to the elbow of the subject, for example. In order to ease pain caused by direct contact of the arm of the subject with securing base main body 11, preferably, a buffer material such as a cushion material made from sponge, for example, is adhered to the surface of concave portion 12. Alternatively, a structure solely having a buffer material adhered without a concave portion in the upper portion of securing base main body 11 may be adopted.

Holding portion 20A is detachably attached to concave portion 11b provided in the upper portion of securing base main body 11. Fastening of holding portion 20A to securing base main body 11 is achieved by a hook and loop fastener 11d adhered to concave portion 11b of securing base main body 11 and a hook and loop fastener 25a adhered to a lower surface of a bottom portion 25 of holding portion 20A (see Fig. 9). In fastening holding portion 20A to securing base main body 11, an engagement protrusion 24 provided in holding portion 20A is fitted into an engagement recess 11c provided in the securing base main body, so as to achieve positioning.

As shown in Figs. 8 and 9, holding portion 20A has support surface 21 receiving the wrist in its upper portion. Support surface 21 is curved so as to fit to the wrist. Support surface 21 includes first support region 27a receiving the radial side portion of the wrist and second support region 27b receiving the ulnar side portion thereof. In a prescribed position of support surface 21 of holding portion 20A, a concave portion 23a receiving the dorsal side portion corresponding to the ulnar styloid process of the left wrist when the left arm is placed on holding portion 20A is provided.

In a prescribed position of a side surface 22 of holding portion 20A, a marker line 28 is provided in an up-down direction. An end of marker line 28 partially enters support surface 21, so that marker line 28 is visually recognized when holding portion 20A is viewed from above. In addition, in a prescribed position of holding portion 20A, engagement protrusion 24 fitted to securing base main body 11 described above is provided.

As shown in Fig. 9, in holding portion 20A, the support surface in first support region 27a is structured to incline more gently than the support surface in second support region 27b. In addition, in holding portion 20A, first support region 27a extends above second support region 27b. Moreover, in holding portion 20A, support surface 21 is formed asymmetrically around vertical centerline 91 including the central portion of support surface 21 in a cross section along a direction in which first support region 27a and second support region 27b are aligned (that is, shown cross section).

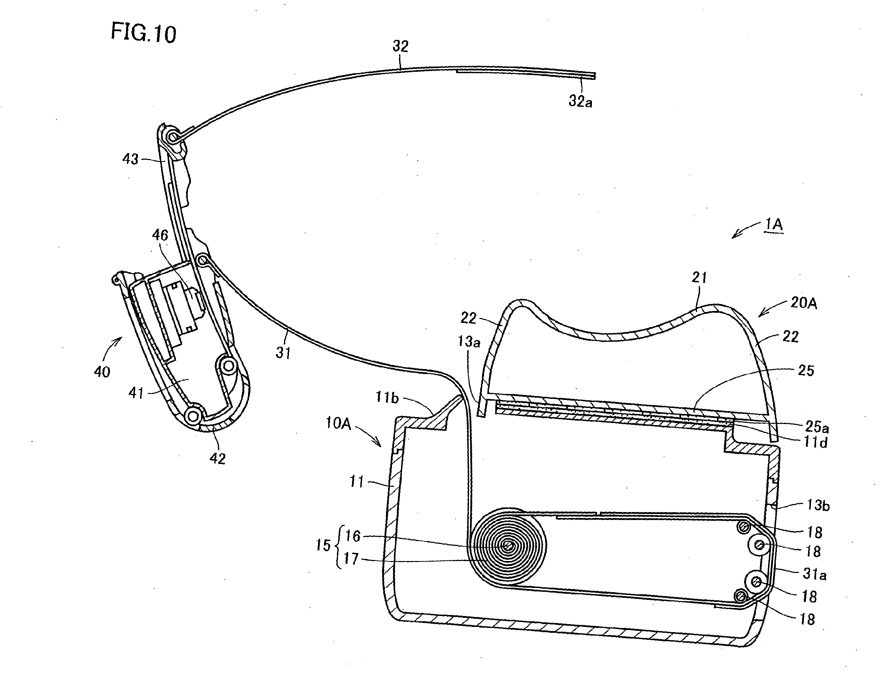

As shown in Fig. 7, sensor unit 40 includes casing body 41 having a pressure-sensing portion 46 (see Fig. 10) and base body 42 supporting casing body 41. Casing body 41 is structured such that it can slide on a rail provided in base body 42 in a longitudinal direction of fastening bands 31 and 32. In other words, casing body 41 freely slides between a position closing opening 43 provided in base body 42 (waiting position) and a position not closing the same (housed position). Fig. 7 shows casing body 41 in a housed position. When casing body 41 is slid, casing body 41 is moved with engagement release button 45 provided on a side surface of casing body 41 being pressed.

Opening 43 is provided such that pressure-sensing portion 46 arranged inside casing body 41 can be pressed against the wrist on which measurement is performed. When pressure-sensing portion 46 is lowered through opening 43, pressure-sensing portion 46 is pressed against the wrist so as to allow measurement of the pulse wave. In addition, display portion 44 for displaying whether or not pressure-sensing portion 46 is in an appropriate position during measurement is provided on the upper surface of sensor unit 40.

As described above, first band portion 31 and second band portion 32 serving as the fastening bands are attached to prescribed positions in base body 42 respectively, with opening 43 of base body 42 of sensor unit 40 lying therebetween. In addition, a hook and loop fastener 32a (see Fig. 10) is adhered on an inner side of a tip end of the other end of second band portion 32.

As shown in Fig. 10, an opening 13a is provided in concave portion 11b provided in the upper portion of securing base main body 11. First band portion 31 is drawn out of opening 13a. In addition, an opening 13b is formed in a side surface of securing base main body 11, through which a portion of first band portion 31 is exposed in its non-attached state (a state in which the wrist is not secured by the living body securing device). Here, a hook and loop fastener (not shown) is adhered to a prescribed region in the side surface adjacent to opening 13b of securing base main body 11. Moreover, in a state in which first band portion 31 is drawn out at the maximum from a wound state by pulling means which will be described later, a hook and loop fastener 31a is adhered also to a surface of a portion exposed through opening 13b.

A constant-load spring 15 serving as the pulling means is arranged inside securing base main body 11. Here, the constant-load spring refers to a spring formed by winding a long-length leaf spring 17 bent with a constant curvature around a shaft 16, and restoring force produced when a tip end of leaf spring 17 is linearly pulled is constantly applied thereto regardless of a pulled length.

First band portion 31 passing through opening 13a has the other end fixed and adhered to the tip end of leaf spring 17. Accordingly, while first band portion 31 is being pulled, the other end of first band portion 31 is constantly pulled by constant-load spring 15 with a constant pull strength.

First band portion 31 is guided by a plurality of rollers 18 in securing base main body 11. Therefore, smooth pull-out and pull-in of first band portion 31 from securing base main body 11 is achieved.

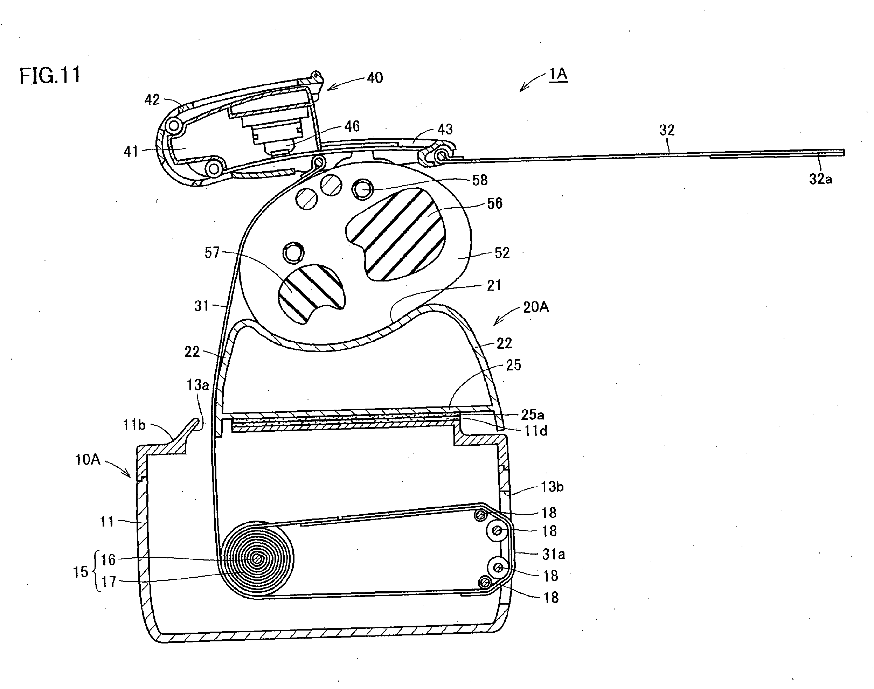

In the following, a procedure for attaching the pulse wave measuring apparatus and a structure after attachment in the present embodiment will be described with reference to Figs. 11 to 13.

First, the arm from the elbow to the wrist is placed on concave portion 12 provided on the upper surface of securing base main body 11. Here, attention is paid such that wrist joint anterior surface stripe 52a located on the body surface on the palm side of the wrist (see Fig. 4) is brought to a position corresponding to positioning marker line 28 provided on the side surface 22 of holding portion 20A. Hence, the wrist can readily be placed on an appropriate position on holding portion 20A. Here, wrist 52 of the subject is held by holding portion 20A of securing base main body 11 in a stable manner, with the end portion on the radial side of wrist 52 being located above the end portion on the ulnar side thereof.

Then, as shown in Fig. 11, first band portion 31 is pulled out from securing base main body 11 by a prescribed length, and sensor unit 40 is brought to a position right above wrist 52 of the subject. Here, the position of radial artery 58 has been checked in advance by palpation or the like, and sensor unit 40 is positioned and arranged such that the center of opening 43 in base body 42 is located approximately above radial artery 58.

Here, as the other end of first band portion 3 1 is fixed and adhered to one end of constant-load spring 15 arranged inside securing base main body 11, a constant pull strength produced by pulling out first band portion 31 from securing base main body 11 is applied to the other end of first band portion 31. Accordingly, when grasping of first band portion 31 is released, an excessive portion of first band portion 31 is pulled back into securing base main body 11 by constant-load spring 15, so that first band portion 31 fits to wrist 52 and securing base main body 11 without loosening. Here, when grasping of first band portion 31 is released, sensor unit 40 should lightly be held on wrist 52 so as to avoid displacement of sensor unit 40 that has been positioned and arranged in advance.

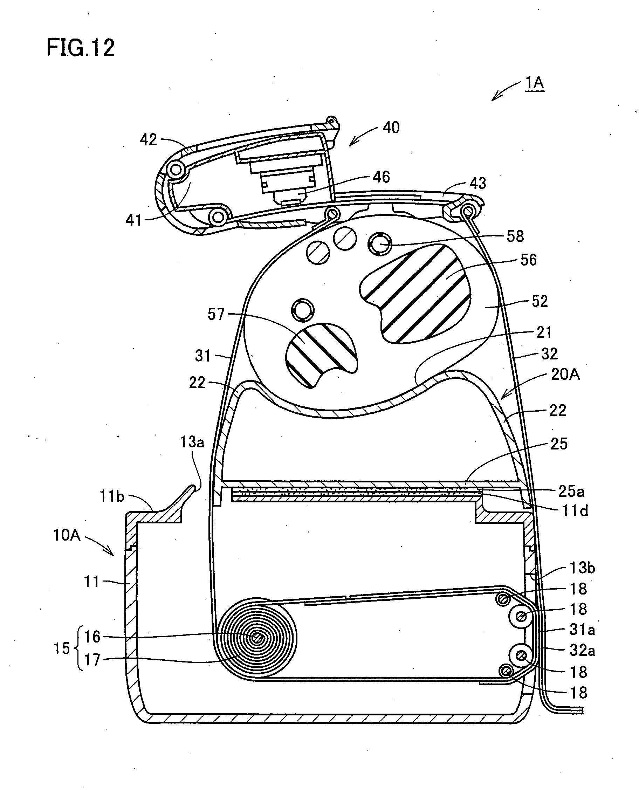

As shown in Fig. 12, second band portion 32 is attached on the side surface of securing base main body 11 on the side opposite to the side surface from which first band portion 31 is pulled out. Here, for attachment of second band portion 32 to securing base main body 11, a hook and loop fastener (not shown) adhered on the side surface of securing base main body 11 and hook and loop fastener 31 adhered to first band portion 31 as well as hook and loop fastener 32a adhered to the inner side of the tip end of the other end of second band portion 32 are used.

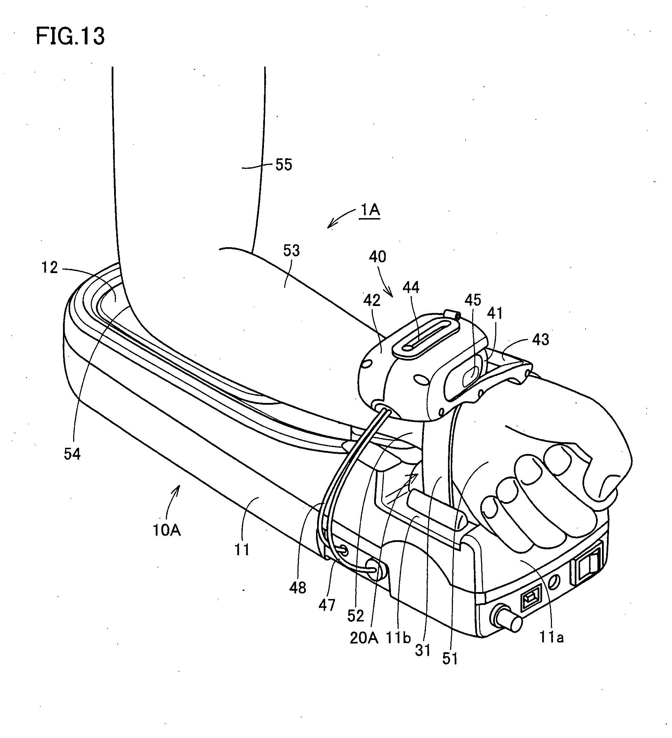

Through the procedure described above, an attached state as shown in Fig. 13 is achieved. In this state, the palm side portion of the left wrist of the subject is slightly inclined toward the body, instead of facing up.

With the above-described structure, when the left arm is placed on holding portion 20A, wrist 52 position can be guided such that the end portion on the radial side of wrist 52 is located above the end portion on the ulnar side thereof. Therefore, a posture for measurement closer to natural for the subject can be achieved, thereby allowing measurement of the pulse wave in a stable manner with high accuracy.

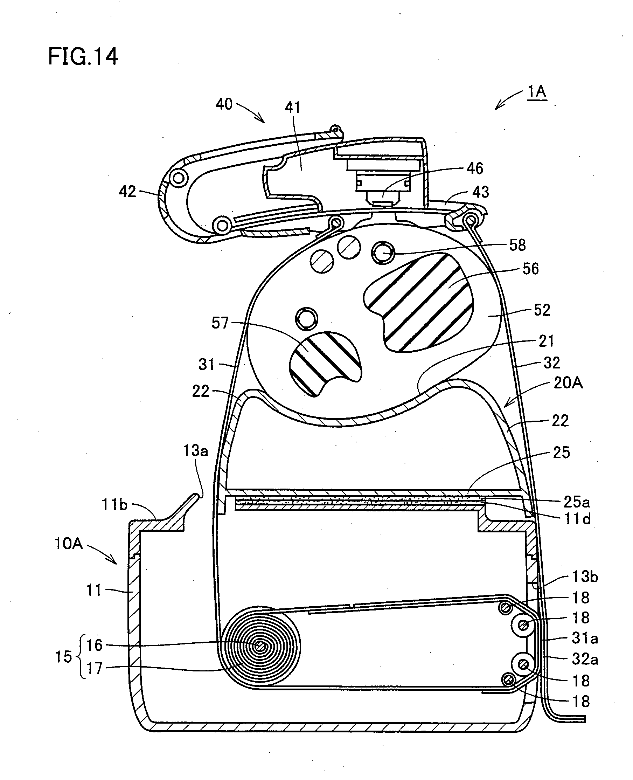

When the pulse wave is actually measured, casing body 41 of sensor unit 40 is slid as shown in Fig. 14, so as to be arranged at a position closing opening 43 in base body 42 (waiting position). Then, an air bag arranged above pressure-sensing portion 46 is expanded using a pressurization pump arranged in securing base main body 11 through air tube 48 (see Fig. 7), whereby pressure-sensing portion 46 is lowered through opening 43 toward wrist 52 and pressed against wrist 52. In this manner, the pulse wave is measured with pressure-sensing means provided in pressure-sensing portion 46.

As described above, in holding portion 20A of wrist securing device 10A for a pulse wave measuring apparatus in the present embodiment, concave portion 23 a is provided in a position facing the dorsal side portion corresponding to the ulnar styloid process of the left wrist (see Fig. 8).

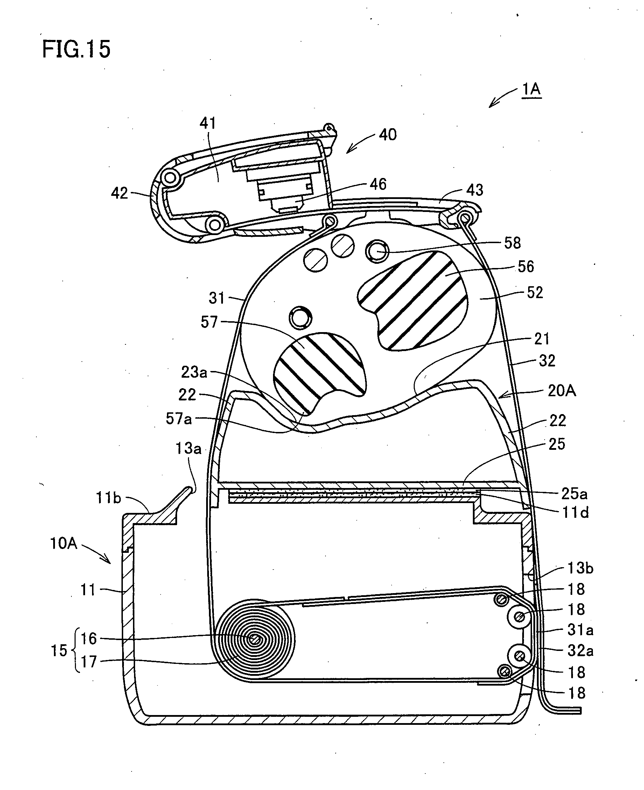

As shown in Fig. 15, while the left arm rests on holding portion 20A, concave portion 23a provided in holding portion 20A receives the dorsal side portion corresponding to the ulnar styloid process of the left wrist. With such a structure, the subject will not move his/her wrist due to pain during measurement from several minutes to over ten minutes, whereby measurement of the pulse wave in a stable manner with high accuracy is allowed.

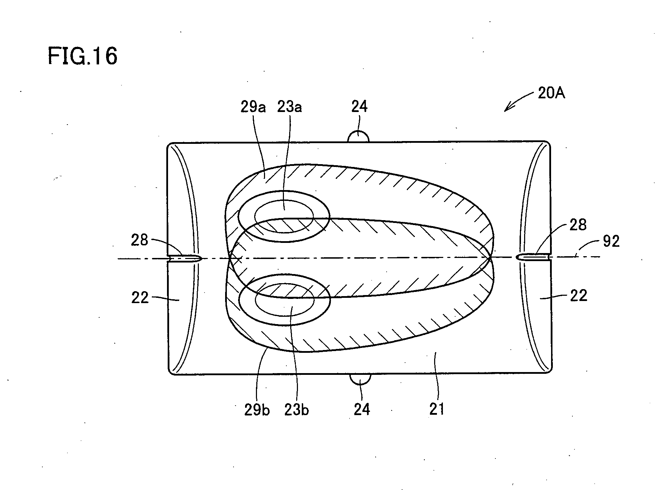

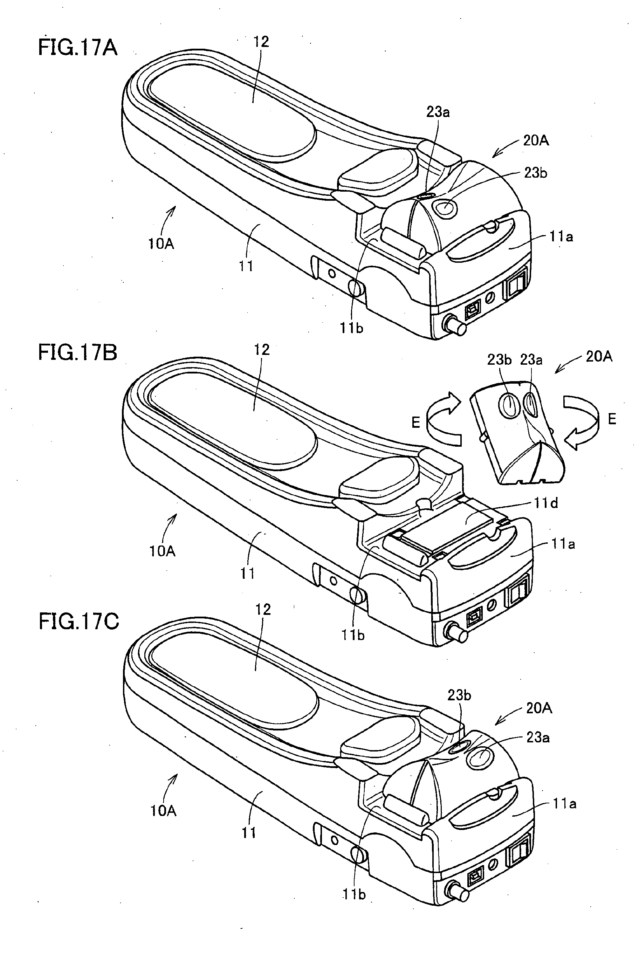

In addition, holding portion 20A of wrist securing device 10A for a pulse wave measuring apparatus in the present embodiment is devised so as to receive also the right arm instead of the left arm. In the following, a structure allowing reception of the right arm instead of the left arm will be described with reference to Figs. 16 and 17A-17C.

As shown in Fig. 16, holding portion 20A is formed symmetrically with respect to a longitudinal direction in the drawing around centerline 92. In other words, holding portion 20A includes a left wrist receiving region 29a receiving the left wrist and a right wrist receiving region 29b receiving the right wrist, that are respectively provided in positions axially symmetrical around centerline 92. Therefore, in addition to concave portion 23a receiving the dorsal side portion corresponding to the ulnar styloid process of the left wrist when the left arm is placed on holding portion 20A, support surface 21 of holding portion 20A includes a concave portion 23b receiving the dorsal side portion corresponding to the ulnar styloid process of the right wrist when the right arm is placed on holding portion 20A, in a position axially symmetrical to concave portion 23 a around centerline 92.

In wrist securing device 10A for a pulse wave measuring apparatus in the present embodiment, holding portion 20A is attached to securing base main body 11 after it is turned by 180° in a plane parallel to the upper surface of securing base main body 11. In this manner, a first state allowing the use of left wrist receiving region 29a and a second state allowing the use of right wrist receiving region 29b are switched. In other words, in the first state allowing the use of left wrist receiving region 29a as shown in Fig. 17A, holding portion 20A is removed from securing base main body 11, and turned in a direction shown with an arrow E as shown in Fig. 17B. Then, holding portion 20A is attached to securing base main body 11 as shown in Fig. 17C, so as to achieve the second state allowing the use of right wrist receiving region 29b.

As described above, wrist securing device 10A for a pulse wave measuring apparatus in the present embodiment can be switched between the use for the right arm and the use for the left arm with a very simple operation, thereby attaining excellent usability.

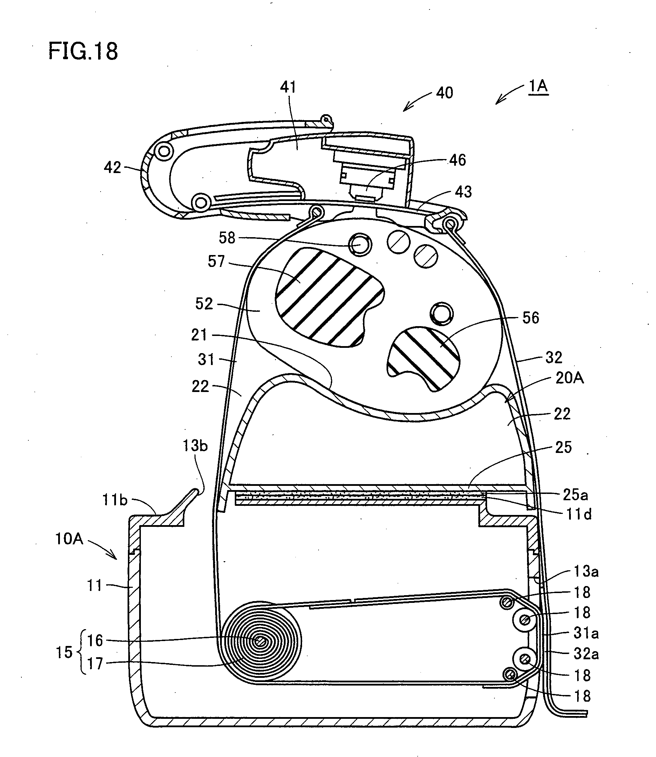

As shown in Fig. 18, in wrist securing device 10A for a pulse wave measuring apparatus in the present embodiment, wrist 52 position is guided such that the end portion on the radial side of wrist 52 is located above the end portion on the ulnar side thereof even while pulse wave measuring apparatus 1A is attached to the right arm of the subject. Therefore, a posture for measurement closer to natural for the subject can be achieved, thereby allowing measurement of the pulse wave in a stable manner with high accuracy.

### [Second Embodiment]

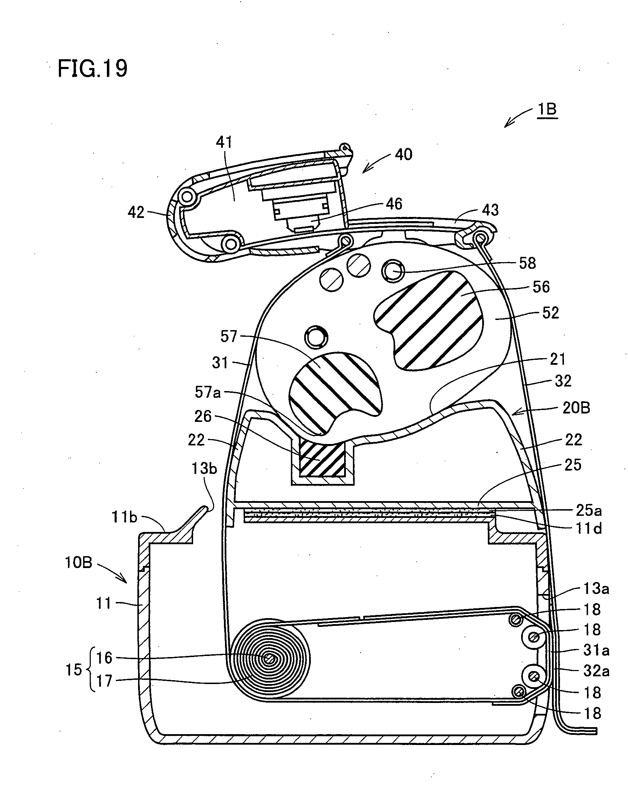

As shown in Fig. 19, a holding portion 20B of a wrist securing device 10B for a pulse wave measuring apparatus in a second embodiment is different from holding portion 20A of wrist securing device 10A for a pulse wave measuring apparatus in the first embodiment described above in a structure of support surface 21 receiving the dorsal side portion corresponding to the ulnar styloid process of wrist 52. As a shown pulse wave measuring apparatus 1B has a structure otherwise the same as pulse wave measuring apparatus 1A in the first embodiment described above, the same reference characters denote the same or corresponding components and description thereof will not repeated.

As shown in Fig. 19, in holding portion 20B of wrist securing device 10B for a pulse wave measuring apparatus in the present embodiment, buffer material 26 is located in a position facing the dorsal side portion corresponding to the ulnar styloid process of the wrist. For example, a sponge-like cushion material formed with synthetic fibers or a rubber material may be employed as buffer material 26.

With such a structure, as a protrusion on the dorsal side portion corresponding to the ulnar styloid process of the wrist is received by deformation of buffer material 26, the subject does not feel pain. Accordingly, the subject will not move his/her wrist due to pain during measurement from several minutes to over ten minutes, whereby measurement of the pulse wave in a stable manner with high accuracy is allowed.

### [Third Embodiment]

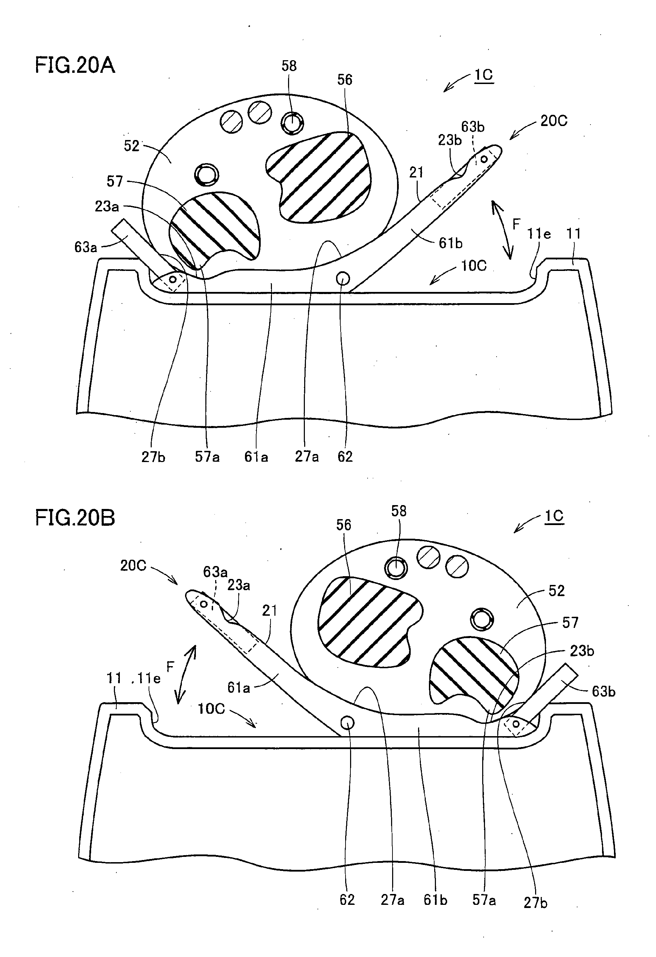

As shown in Figs. 20A and 20B, a holding portion 20C of a wrist securing device 10C for a pulse wave measuring apparatus in a third embodiment is different from holding portion 20A of wrist securing device 10A for a pulse wave measuring apparatus in the first embodiment described above in a structure for attachment to the securing base main body. As a shown pulse wave measuring apparatus 1 C has a structure otherwise the same as pulse wave measuring apparatus 1A in the first embodiment described above, the same reference characters denote the same or corresponding components and description thereof will not repeated.

In wrist securing device 10C for a pulse wave measuring apparatus in the present embodiment, unlike wrist securing devices 10A and 10B for a pulse wave measuring apparatus in the first and second embodiments, holding portion 20C is attached to an upper portion of securing base main body 11 in a swingable manner by means of a swing shaft 62, instead of being attached to securing base main body 11 in a detachable manner. Here, swing of holding portion 20C is restricted to be within a certain range by abutment of the lower surface of holding portion 20C on the bottom surface of a concave portion 11e provided in the upper portion of securing base main body 11.

Holding portion 20C includes a first base portion 61a and a second base portion 61b extending in different directions from swing shaft 62. A pivot member 63a is pivotably attached to an end portion of first base portion 61a, and a pivot member 63b is pivotably attached to an end portion of second base portion 61b. In addition, concave portion 23a receiving the dorsal side portion corresponding to the ulnar styloid process of the left wrist is provided in a prescribed position in first base portion 6 1a, and concave portion 23b receiving the dorsal side portion corresponding to the ulnar styloid process of the right wrist is provided in a prescribed position in second base portion 61b.

Fig. 20A shows a first state in which the left wrist rests on holding portion 20C. In this state, the lower surface of first base portion 61a of holding portion 20C abuts on the bottom surface of concave portion 11e provided in securing base main body 11, and swing of holding portion 20C is restricted by a not-shown stopper. Support surface 21 including left wrist receiving region 29a receiving left wrist 52 is implemented by the upper surface of first base portion 61a and the upper surface of pivot member 63a. Support surface 21 has first support region 27a receiving the radial side portion of wrist 52 and second support region 27b receiving the ulnar side portion of wrist 52. Support surface 21 has a shape receiving wrist 52 in an inclined position such that the end portion on the radial side of wrist 52 is located above the end portion on the ulnar side thereof.

Fig. 20B shows a second state in which the right wrist rests on holding portion 20C. In this state, the lower surface of second base portion 61b of holding portion 20C abuts on the bottom surface of concave portion 11e provided in securing base main body 11, and swing of holding portion 20C is restricted by a not-shown stopper. Support surface 21 including right wrist receiving region 29b receiving right wrist 52 is implemented by the upper surface of second base portion 61b and the upper surface of pivot member 63b. Support surface 21 has first support region 27a receiving the radial side portion of wrist 52 and second support region 27b receiving the ulnar side portion of wrist 52. Support surface 21 has a shape receiving wrist 52 in an inclined position such that the end portion on the radial side of wrist 52 is located above the end portion on the ulnar side thereof.

The first state shown in Fig. 20A and the second state shown in Fig. 20B are switched by an operation of the not-shown stopper and swing of holding portion 20C in a direction shown with arrow F around swing shaft 62. In other words, when holding portion 20C swings like a seesaw, the first state allowing the use of left wrist receiving region 29a and the second state allowing the use of right wrist receiving region 29b are switched.

With such a structure, the wrist securing device for a pulse wave measuring apparatus can be switched between the use for the right arm and the use for the left arm with a very simple operation, thereby attaining excellent usability.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A wrist securing device for a pulse wave measuring apparatus, comprising:
a securing base main body (11) mounted on a mount surface (80); and
a holding portion (20A to 20E) provided in an upper portion of said securing base main body (11) and receiving a wrist (52) with a palm side portion of the wrist (52) facing upward, said wrist securing device for a pulse wave measuring apparatus holding a position of the wrist (52) placed on said holding portion (20A to 20E) for measuring periodic fluctuation of an intra-arterial pressure of an radial artery (58) located under skin of the wrist (52); wherein
said holding portion (20A to 20E) has a support surface (21) receiving a dorsal side portion of the wrist (52) while the wrist (52) rests on the holding portion (20A to 20E),
said support surface (21) includes a first support region (27a) receiving a radial (56) side portion of the wrist (52) and a second support region (27b) receiving an ulnar (57) side portion of the wrist (52), and
said support surface (21) is asymmetrical around a vertical centerline (91) including a central portion of the support surface (21) in a cross section along a direction in which said first support region (27a) and said second support region (27b) are aligned.

2. The wrist securing device for a pulse wave measuring apparatus according to claim 1, wherein
said holding portion (20A to 20E) has a concave portion (23a, 23b) in a position corresponding to an ulnar styloid process (57a) of the wrist (52) while the wrist (52) rests on the holding portion (20A to 20E).

3. The wrist securing device for a pulse wave measuring apparatus according to claim 1, wherein
said holding portion (20A to 20E) has a buffer portion (26) composed of an elastic member in a position corresponding to an ulnar styloid process (57a) of the wrist (52) while the wrist (52) rests on the holding portion (20A to 20E).

4. The wrist securing device for a pulse wave measuring apparatus according to claim 1, wherein
said holding portion (20A to 20E) has a right wrist receiving region (29b) receiving right wrist (52) and a left wrist receiving region (29a) receiving left wrist (52).

5. The wrist securing device for a pulse wave measuring apparatus according to claim 4, wherein
said holding portion (20A to 20E) is structured to be switchable between a first state allowing use of said right wrist receiving region (29b) and a second state allowing use of said left wrist receiving region (29a).

6. The wrist securing device for a pulse wave measuring apparatus according to claim 5, wherein
said holding portion (20A to 20E) is attached to said securing base main body (11) in a detachable manner.

7. The wrist securing device for a pulse wave measuring apparatus according to claim 5, wherein
said holding portion (20A to 20E) is attached to said securing base main body (11) in a swingable manner.

8. The wrist securing device for a pulse wave measuring apparatus according to claim 1, wherein
said holding portion (20A to 20E) has a positioning marker (28) in a position corresponding to wrist joint anterior surface stripe (52a) located on an outer surface of the wrist (52) while the wrist (52) rests on the holding portion (20A to 20E).

9. A pulse wave measuring apparatus, comprising:
a sensor unit (40) having a pressure-sensing portion (46); and
a living body securing device holding a position of a wrist (52), said pulse wave measuring apparatus serving to measure a pulse wave by pressing said pressure-sensing portion (46) against the wrist (52) while the wrist (52) is secured by said living body securing device; wherein
said living body securing device includes the wrist securing device for a pulse wave measuring apparatus according to claim 1.
